# EUROPEAN PATENT SPECIFICATION

(11) **EP 3 082 765 B1**
(45) Date of publication and mention of the grant of the patent: **01.08.2018**
(21) Application number: 14821857.1
(22) Date of filing: 17.12.2014
(51) Int. Cl.: A61K 9/06, A61K 47/26, A61K 47/36

(54) **DRUG DELIVERY SYSTEMS**
WIRKSTOFFFREISETZUNGSSYSTEME
SYSTÈMES D'ADMINISTRATION DE MÉDICAMENTS

(30) Priority: 19.12.2013 US 201361918378 P
(43) Date of publication of application: 26.10.2016
(73) Proprietor: Novartis AG, 4056 Basel (CH)
(72) Inventor: SUPPER, Stephanie, CH-4002 Basel (CH)
(74) Representative: Petersen, Holger
(86) International application number: PCT/IB2014/066986
(87) International publication number: WO 2015/092690

(56) References cited:
- WO-A1-99/07416
- US-A1- 2008 248 991
- CHENITE A ET AL: "Novel injectable neutral solutions of chitosan form biodegradable gels in situ", BIOMATERIALS, ELSEVIER SCIENCE PUBLISHERS BV., BARKING, GB, vol. 21, no. 21, 1 November 2000 (2000-11-01), pages 2155-2161, XP004216030, ISSN: 0142-9612, DOI: 10.1016/S0142-9612(00)00116-2
- CHEN XINGWEI ET AL: "Chitosan-based thermosensitive hydrogel as a promising ocular drug delivery system: preparation, characterization, and in vivo evaluation", JOURNAL OF BIOMATERIALS APPLICATIONS,, vol. 27, no. 4, 1 November 2012 (2012-11-01), pages 391-402, XP008175115, ISSN: 1530-8022, DOI: 10.1177/0885328211406563 cited in the application

## Description

### FIELD OF INVENTION

The present invention relates to thermogelling peptide delivery systems which are injected as fluid sol at ambient temperature through thin injection needles and transform into a highly viscous gel once inserted into human or animal body warm tissue. The peptide is then released from the thermally-induced hydrogel into the surrounding tissue in a controlled manner over a longer period of time.

### BACKGROUND OF THE INVENTION

Many pharmaceutically active peptides cannot be delivered effectively via oral administration as most peptides are prone to degradation in the gastrointestinal tract. As consequence, those peptides need to be delivered parenterally. However, parenteral administration of the peptoid drug into the subcutis or muscle tissue is associated with patient's discomfort by needle injections. Those invasive injections need to be repeated quite frequently as many peptides have only a short half life of a few hours or even less than one hour. Parenteral depot formulations in the form of implants or microparticle suspensions have therefore been delevoped to reduce the frequency in which those injections have to occur.

For the comfort of the patient suffering from a chronic disease, there is a need to further develop today's available depot formulation technologies towards sustained drug delivery systems which can be injected through very thin needles.

Thermally-induced hydrogels which can be injected as fluid sol and which solidify as very viscous hydrogel were suggested. However, many of those hydrogels are based on high molecular weight polymers which, once dissolved in aqueous media, turn into highly viscous solutions so that again a large needle size is required for the injection (e.g. in X. Chen et al., J. Biomat. Applications 2012, 27(4), 391-402, because of the use of a high MW (500 kDa) chitosan the sol had to be instilled into the eye using a needleless syringe). A further issue with the so far proposed systems is a very strong drug release short after the injection (the so-called "burst") so that a significant amount of the drug is already released into the tissue immediately before the fluid transforms into a hydrogel. Even further, some systems have a stability issue and transform into a hydrogel already during storage time which makes them unusable for a later injection (e.g. as observed by the inventor of the present invention for chitosan / beta-glycerophosphate systems as described earlier e.g. in WO99/07416).

### SUMMARY OF THE INVENTION

Therefore, there is still a need to a sustained peptide drug delivery system which fulfills all the following requirements to minimize patients' discomfort and to maximize comfort, safety and operability for the injection performed by the patient himself or by clinical practitioners:
- Ready-to-use (the drug product immediatedly ready for to be injected, no significant preparatory efforts have to be made, e.g. it is not required that a dry powder is to be reconstituted with an aqueous vehicle to get to an injectable suspension)
- Injectability through a very thin needle (e.g. 21 G or smaller)
- Sustained drug release over a long time period (e.g. one or more months)
- Biocompatability (e.g. non of the components causes pain after injection)
- Biodegradability (e.g. all components are completely resorbed by the body after reasonable time to avoid accumulation of any component after repetitive injection events)
- Physical stability over reasonable shelf life (e.g. no chance of sol quality of the stored system over time during storage under cool conditions or at room temperature)
- Good safety profile of excipients

The present invention surprisingly meets all those requirements and is therefore a commercially viable peptide delivery system.

In a first aspect, the present invention provides a drug delivery system comprising:
(a) a pharmaceutically active peptide or any pharmaceutically acceptable salt thereof, e.g. pasireotide pamoate,
(b) 1 to 2% of weight of a chitosan (CS) or chitosan derivative, having a deacylation degree (DD) from 85 to 95% and a molecular weight (MW) from 100 to 200 kDa,
(c) 10 to 25% of weight of a sugar-phosphate, preferably a glucose-1-phosphate (G1-P) and
(d) water, based on the total weight of the drug delivery system.
In a second aspect, the present invention provides a process for preparing the drug delivery system as defined by the first aspect comprising following process steps:
(1) dissolving the chitosan in an acidic aqueous solution, preferably in a hydrochloric acid solution,
(2) dissolving the sugar-phosphate in an aqueous medium, preferably in water,
(3) dissolving, partly dissolving, or dispersing the peptide into the sugar-phosphate solution, preferably dispersing the peptide, and
(4) combining the peptide/glucose-phosphate solution and/or suspension with the chitosan solution.
The drug delivery system according to the first aspect is for intramuscular or subcutaneous use, preferably for subcutaneous use.

In a third aspect, there is provided to a dosage form comprising the drug delivery system according to the first aspect in the form of a liquid in an ampoule, vial or pre-filled syringe.

### BRIEF DESCRIPTION OF THE DRAWINGS

Fig. 1 shows the pasireotide release profiles from CS / G1-P hydrogels versus CS solutions (mean ± SD, n = 3) and demonstrates the controlled drug release of the peptide in contrast to a control.
Fig. 2 shows the pasireotide release profiles from CS / G1-P hydrogels as a function of initial loading (mean ± SD, n = 3).
Fig. 3 shows the apparent shear viscosity at 20.0 ± 0.2°C for CS solution and CS / gelling agent systems stored at 2 - 8°C and at 20 - 25°C (n = 3) and demonstrates the good physical stability of the embodiments of the present invention in contrast to a reference embodiment.
Fig. 4 shows the evolution of the gelation time, determined by rheology at 37°C, of CS / gelling agent solutions stored at 2 - 8°C and at 20 - 25°C (n = 3) which demonstrates the good physical stability of the embodiments of the present invention in contrast to a reference embodiment.
Fig. 5 shows the injection forces of 1 mL CS / G1-P solution (1.5 wt.% CS and 0.40 mmol/g G1-P) injected in air at two injection speeds with 23 G, 25 G and 30 G needles (n = 10) and demonstrates the good injectability of the embodiments of the present invention.
Fig. 6 shows the relative Mw of CS for three different batches of 1.5 wt.% CS / 0.40 mmol/g G1-P solution non-filtered and sterilized by filtration through a 0.2 µm membrane (n = 3) and demonstrates the feasibility to sterilize the embodiments of the present invention to make them suitable for parenteral use.

### DETAILED DESCRIPTION OF THE INVENTION

Herein after, the present invention is described in further detail and is exemplified.

In the aspects of the present invention the peptide is preferably hydrophobic. In one embodiment the peptide is hydrophobic by means of the characteristics of its aminoacids. In another embodiment the peptide is hydrophobic by means of its counterion(s) which make the peptide as salt hydrophobic, e.g. salts like dibunate, naphtoate, octadecanoate, oleate, oxalate, palmitate, pamoate/embonate, stearate, xinafoate. In a preferred embodiment the peptide is present as pamoate salt, more preferably as monopamoate salt.

In a preferred embodiment the hydrophobic peptide or the salt thereof is present in an amorphous form (degree of crystallinity < 20%, preferably < 5%) and micronized (e.g. by jet milling) to a particle size disdribution (by laser light diffraction) of x90 <= 10 µm, e.g. 3 µm, x50 <= 5 µm, e.g. 1 µm, x10 >= 0.3 µm, e.g. 0.7 µm and posseses a specific surface area (by BET) >= 8 m²/g, e.g. 32 m²/g).

In yet another preferred embodiment the peptide is a somatostatin analog, e.g. octreotide, pasireotide, vapreotide, or lanreotide, preferably octreotide or pasireotide, more preferably pasireotide. In a preferred embodiment the hydrophobic peptide is octreotide pamoate or pasireotide pamoate.

"Hydrophobic" herein is referred to as a characteristics of a drug substance which is sparingly, slightly, or very slightly soluble in water at ambient temperatures (e.g. 20 - 25 °C) in accordance to Pharmacopeia's (USP and PhEur) descriptive terms for solubility with its solubility ranges of 10 - 33, 1 - 10, or 0.1 - 1 g/L, respectively. Preferably the hydrophobic peptide is very slightly soluble in water at ambient temperature, preferably the water solubility is approximately 1 g/L or lower at ambient temperatures. E.g. hydrophobic peptides are pasireotide pamoate with a solubility in water of 0.02 g/L or octreotide pamoate with a solubility in water of 0.2 g/L (25°C) or vapreotide pamoate with a solubility in phosphate buffer saline (PBS pH 7.4) of about 0.02 - 0.04 g/L.

In the aspects of the present invention a chitosan or a chitosan derivative (e.g. chitosan HCl, carboxymethylchitosan, chitosan lactate, chitosan acetate) is used. A chitosan is used which has a deacylation degree (DD) from 85 to 95 %, preferably a DD of 90 ± 2.5 %. A chitosan is used which has a molecular weight (MW) from 100 to 200 kDa, preferably a MW from 100 to 150 kDa, more preferably a MW of 135 ± 20 kDa.

Chitosans are obtainable in e.g. by Heppe Medical Chitosan GmbH, Halle (Saale), Germany, e.g. in the type of Chitosan 90/10 (DD 87.6 - 92.5%, Viscosity 8 - 15 mPas, corresponds to a MW (GPC) of ca. 20 - 100 kDa), Chitosan 90/20 (DD 87.6 - 92.5%, Viscosity 16 - 30 mPas, corresponds to a MW (GPC) of ca. 40 - 150 kDa), Chitosan 90/50 (DD 87.6 - 92.5%, Viscosity 31 - 70 mPas, corresponds to a MW (GPC) of ca. 80 - 200 kDa), Chitosan 90/100 (DD 87.6 - 92.5%, Viscosity 71 - 150 mPas, corresponds to a MW (GPC) of ca. 100 - 250 kDa), or Chitosan 90/200 (DD 87.6 - 92.5%, Viscosity 151 - 350 mPas, corresponds to a MW (GPC) of ca. 150 - 300 kDa). The viscosities are measured as 1 % solution of the chitosan in 1% acetic acid at 20°C. In a preferred embodiment of the present invention a Chitosan 90/50 is used.

In the aspects of the present invention a sugar-phosphate is used. In the embodiments of the presente invention the sugar of said sugar-phosphate is a monosaccharide, disaccharide, or a oligosaccharide, preferably a monosaccharides selected from the group of aldoses, e.g. glucose, mannose, galactose, allose, altrose, gulose, idose, talose, ribose, arabinose, xylose, lyxose, or from the group of ketoses, e.g. fructose, sorbose, tagatose, psicose, araboketose, xyloketose, or a mixture thereof, preferably glucose or fructose or a mixture thereof.

In a preferred embodiment said sugar-phosphate is a glucose-phosphate, preferably a glucose-1-phosphate, preferably a alpha-D-glucose-1-phosphate, more preferably alpha-D-glucose-1-phosphate disodium hydrate.

In the aspects of the present invention the drug delivery system comprises at least 1 %, at least 2%, 1 to 30 %, 2 to 10 %, or 2 to 5 %, preferably 2 to 5 % by weight of the peptide in its free base form based on the total weight of the drug delivery system. The drug delivery system comprises 10 - 25 % by weight of the sugar-phosphate of the drug delivery system based on the total weight of the drug delivery system. Preferred embodiments comprise 10, 15, and 20 % by weight of the sugar-phosphate glucose-1-phosphate based on the total weight of the drug delivery system. In those preferred embodiments the percentage values correspond to 0.27, 0.40 and 0.53 mmol/g, respectively.

In a preferred embodiment the drug delivery system comprises, substantially comprises, essentially consists of, or consists of, preferably consists of
(a) 2 to 5 % by weight of a hydrophobic peptide in its free base form, preferably present as pamoate salt, preferably the petide is pasireotide pamoate,
(b) 1 to 2 % by weight of a chitosan having a DD of 90 ± 2.5 % and a MW of 135 ± 20 kDa, subjected to an acid, preferably subjected to hydrochloric acid.
(c) 10 - 25 % by weight of glucose-1-phosphate, and
(d) water.
based on the total weight of the drug delivery system

In a second aspect of the present invention there is provided a process for preparing the drug delivery system as defined by the first aspect comprising following process steps:
(1) dissolving the chitosan in an acidic aqueous solution, preferably in a hydrochloric acid solution,
(2) dissolving the sugar-phosphate in an aqueous medium, preferably in water,
(3) dissolving, partly dissolving, or dispersing the peptide into the sugar-phosphate solution, preferably dispersing the peptide, and
(4) combining the peptide/glucose-phosphate solution and/or suspension with the chitosan solution.

In a preferred embodiment the solutions resulting from steps (1) and (2) are cooled, e.g. to 1 - 12 °C, preferably to 2 - 8 °C, before further processed according to steps (3) and (4).

In a preferred embodiment step (3) comprises the use of ultrasound and is performed under cool conditions. Cool conditions are e.g. conditions which ensure that the components involved those step (3) are kept in the range of 1 - 12 °C, preferably in the range of 2 - 8 °C during the ultrasound treatment and thereafter.

In a preferred embodiment in step (4) the peptide/glucose-phosphate solution is added to the chitosan solution under cool conditions. Cool conditions are e.g. conditions which ensure that the components involved step (4) are kept in the range of 1 - 12 °C, preferably in the range of 2 - 8 °C during the addition and thereafter.

In a third aspect, there is provided a drug delivery system obtainable by the process according to second aspect.

In the fourth aspect of the present invention the drug delivery system according to the first or third aspect is for intramuscular or subcutaneous use. In a preferred embodiment the drug delivery system is for subcutaneous use. In a preferred embodiment the drug delivery system is delivered into the subcutis via a 21G, 23G, 25G, or 30G needle, preferably via a 25 needle.

In the fifth aspect of the present invention the dosage form comprising the drug delivery system according to the first, third, or forth aspect is in the form of a liquid in an ampoule, vial or pre-filled syringe, preferably in a pre-filled syringe. In a prefered embodiment the pre-filled syringe is equipped with a 21G, 23G, 25G, or 30G needle, preferably via a 25 needle.

As a preferred embodiment the drug delivery system according to the the first, third, fourth, or fifth aspect is in the form of a fine and homogeneous suspension comprising peptide or peptide salt particles of which 90% or more are smaller than 30 µm chord length (measured by focussed beam reflectance measurements, FBRM, e.g. with a laser light scanning probe by Lasentec).

Said suspension is stable enough so that it can be filled into ampoules, vials or pre-filled syringes and need only to be shaken gently after long storage time short before use.

### EXAMPLES

### Materials:

Chitosan (CS) as used in the following examples is commercially available e.g. from Heppe Medical Chitosan (HMC, Halle, Germany) in technical grade, e.g. Chitoscience® 90/50, or in pharmaceutical grade, e.g. Chitoceutical® 90/50 (Batch 1) and Chitoceutical® 90/50 (Batch 2). The viscosity of those chitosans is measured with a viscosimeter (Brookfield DVI+pro) at 20°C (1% in 1% acetic acid) and the viscosity data are in the range of 31 - 70 mPas. The DD is measured either by photometry or by titration and the DD data are in the range of 87.6 - 92.5 %. The MW is determined by size exclusion chromatography (SEC) in combination with refractive index (RI) detection and the MW data are in the range of 80 - 200 kDa. Alpha-D-glucose-1-phosphate disodium salt hydrate (G1-P, Mw: 376.16 g/mol) and hydrochloric acid (HCl) is commercially available from various sources. The synthesis of the peptide pasireotide has been described before, e.g. in WO02/10192. Purified water was used, e.g. Ultrapure® water obtainable via a MilliQ® Millipore filtration system (Millipore, Molsheim, France).

### Physical characterisation of chitosans:

The CS used for the present invention were characterized by triple detection gel permeation chromatography (GPC) on a Viscotek Triple Detector Array max system (Viscotek, USA) using two ViscoGel A6000M (mixed Bed) columns (Malvern Instruments GmbH, Germany). The set up consisted of a size exclusion chromatograph connected to a light scattering cell; a refractive index detector and a viscometer, allowing for simultaneous determination of the absolute polymer molecular weight (Mw), hydrodynamic radius and intrinsic viscosity (η). The analysis were performed at 30°C with a 0.3 M acetic acid, 0.3 M sodium acetate, 1 % ethylene glycol mobile phase and using a flow rate of 0.7 mL/min and a dn/dc of 0.163 mL/g. CS samples were prepared at concentrations of 1 mg/mL, dissolved 24 h under agitation in the same buffer and then filtered using a 0.2 µm filter prior to analysis. The injection volume was 100 µL. Calibration of the system was made using a Pullulan standard (Mw = 133 kDa, [η] = 0.515 dL/g, polydispersity index Mw/Mn = 1.08) obtained from Viscotek. For the evaluation of the physical data OmniSEC software from Viscotek was used. Each characterization of CS samples was performed in duplicate.

Results are reported in Table 1.

**Table 1: Physical parameters obtained for the different chitosan batches.**

| Name | Deacylation degree (%) (supplier information) | Absolute Mw (kDa) | Mw/Mn | Intrinsic viscosity (dL/g) | Hydrodynamic radius (nm) |
|---|---|---|---|---|---|
| Chitoscience® 90/50 | 89.7 | 122 | 1.57 | 3.62 | 18.33 |
| Chitoceutical® 90/50(1) | 90.7 | 135 | 1.61 | 3.86 | 19.28 |
| Chitoceutical® 90/50(2) | 88.7 | 149 | 1.62 | 4.11 | 20.30 |
| Chitoscience® 90/200 | | 267 | 1.90 | 6.01 | 27.74 |

### Preparation of the formulations

### CS solution:

CS solutions were obtained by dissolving 3.75 wt.% CS in hydrochloric acid (HCl) according to a molar ratio for chitosan amine groups : HCI equal to 0.9 : 1.

### G1-P solution:

G1-P solution was prepared at 350 mg.mL-1 in MilliQ water.

### Peptide loaded CS / G1-P suspensions and particle size determination:

The peptide pasireotide pamoate was added into the G1-P solution, dispersed by magnetic stirring for 2 h and sonicated 2 min using an ultrasound probe (amplitude 80 %, cycle 1, Hielscher Ultrasound technology UP400S Ultrasonic processor, Stuttgart, Germany) under magnetic stirring in an ice-bath. 3.60 g G1-P / peptide suspension was then added dropwise into 2.40 g of cold CS solution under magnetic stirring in an ice bath. The obtained CS / G1-P / peptide formulation was further stirred until a homogeneous suspension was obtained, as verified by optical microscopy and focused beam reflectance measurements (FBRM) (Table 2). The final formulations, containing 1.5 wt.% CS, 0.40 mmol/g G1 P and 2.5 or 5.0 wt.% peptide, were stored at 2 - 8°C until use.

FBRM data were obtained using a Lasentec FBRM PI-14/206 probe (Mettler-Toledo, USA.) and analyzed with Lasentec FBRM software (Version 6.7.0 09/2005 Mettler Toledo, Columbia, USA). This light scattering technique measures the backscattered light from a rotating laser beam focused outside a sapphire window, in contact with the suspension. When the laser light scans particles near the probe window, it generates backscattered light signals whose duration is translated into chord lengths (straight-line distance from one edge of the particle to another). Therefore, even if the chord length is related to the particle size, it does not measure directly the particle size distribution, but rather the chord length distribution.

**Table 2: Chord length distribution in the CS / G1-P / peptide suspensions**

| Formulation | Peptide content (wt.%) | Median square-weighted chord length (µm) | Mean square-weighted chord length (µm) | < 30 µm (%) |
|---|---|---|---|---|
| **Example 1**: | 2.50 | 10.27 | 12.66 | 94.56 |
| CS / G1-P / Peptide2.5 | | | | |
| **Example** 2**:** | 5.00 | 10.87 | 12.57 | 96.61 |
| CS / G1-P / Peptide5 | | | | |

### Peptide loaded CS suspensions (Control without glucose-phosphate):

CS / Peptide suspensions as controls were prepared following the same protocol but dispersing the peptide in water instead of G1-P solution.

Detailed description of preparation method (Batch size 6 g) for CS/G1-P/pasireotide 2.5% **(Example 1)** and CS/G1-P/pasireotide 5% **(Example 2):**

### Materials:

Chitoceutical 90/50 by HMC DD: 88.7% Mw: 149 kDa; HCl 1 M by Fluka; G1-P disodium by Sigma, Mw: 361.59 g/mol; Pasireotide pamoate, amorphous, micronized (Mw free base: 1047.21, Mw pamoate salt: 1435.53, particle size disdribution by laser light diffraction: x90 <= 10 µm, here: 3 µm, x50 <= 5 µm, here: 1 µm, x10 >= 0.3 µm, here 0.7 µm, specific surface area (BET) >= 8 m²/g, here 32 m²/g); Water (MilliQ).

### (1) Preparation of chitosan solution

40.00 g of 3.75 % w/w chitosan solution in HCI 0.185M was prepared by magnetic stirring overnight. The chitosan solution is filtered through a 11 µm nylon filter to remove any insoluble particulate matter. The solution is stored at 2 - 8 °C.

| | Required | Actual |
|---|---|---|
| m_{CS}(g) | 1.50 | 1.50 |
| HCl 0.185M qsp (g) | 40.00 | 40.00 |
| C_{CSsol} (mg/g) | 37.5 | 37.5 |
| pH | 5.5-5.8 | 5.55 |

### (2) Preparation of CS/G1-P/pasireotide 2.5% thermogelling suspensions (Example 1):

### (2.1) Preparation of G1-P/pasireotide pamoate 5.712% suspension:

342.70 mg pasireotide pamoate and 1500.00 mg G1-P is weight into a glass beaker, and adjusted to 6.000 g with water. The mixture is pre-homogenized by magnetic stirring for 2h. Then, the mixture is homogenized by sonication for 5 min using an ultrasound probe (amplitude 80 %, cycle 1) under magnetic stirring in an ice-bath.

| | Required | Actual |
|---|---|---|
| m_{G1-P} (mg) | 1500.0 | 1500.11 |
| m_{pasireotide pamoate} (mg) | 342.70 | 342.29 |
| H₂O qsp (g) | 6.000 | 5.9987 |
| C_{G1-P} (mg/g) | 250.00 | 250.07 |
| C_{pasireotide pamoate} (mg/g) | 57.12 | 57.06 |

### (2.2) Preparation of CS/G1-P/SOM230 2.5% thermogelling suspension:

2.400 g chitosan solution is weight into a tared glass flask with a magnetic stirrer. In an ice-bath, the chitosan solution and the G1-P/pasireotide pamoate 5.712 % suspension is chilled for at least 15 min. Drop by drop 3.600 g G1-P/pasireotide pamoate 5.712 % suspension is added to the chitosan solution under magnetic stirring. Stirring of the obtained suspension is continued for at least 30 min or until the drug substance is thoroughly dispersed. The pH and the mean particle size (using a Lasentec probe, FBRM) is determined. The suspension is stored at 2 - 8°C.

| | Required | Actual |
|---|---|---|
| m_{CSsol} (g) | 2.400 | 2.40039 |
| m_{G1-P/pasireotide pamoate 5.712%} (g) | 3.600 | 3.60797 |
| m_{CS/G1-P/pasireotide 2.5%} (g) | 6.000 | 6.00835 |

### (3) Preparation of CS/G1-P/Pasireotide 5% thermogelling suspensions (Example 2):

### (3.1) Preparation of G1-P/pasireotide pamoate 11.423% suspension:

685.40 mg pasireotide pamoate and 1500.00 mg G1-P are weight into a glass beaker and adjusted to 6.000 g with water. The mixture is pre-homogenized by magnetic stirring for 2h. Then, the mixture is homogenized by sonication for 5 min using an ultrasound probe (amplitude 80 %, cycle 1) under magnetic stirring in an ice-bath.

| | Required | Actual |
|---|---|---|
| m_{G1-P} (mg) | 1500.0 | 1499.81 |
| m_{pasireotide pamoate} (mg) | 685.40 | 685.60 |
| H₂O qsp (g) | 6.000 | 5.99967 |
| C_{G1-P} (mg/g) | 250.00 | 249.98 |
| C_{pasireotide pamoate} (mg/g) | 114.23 | 114.27 |

### (3.2) Preparation of CS/G1-P/Pasireotide 5% thermogelling suspension:

2.400 g chitosan solution is weight into a tared glass flask with a magnetic stirrer. In an ice-bath, the chitosan solution and the G1-P/SOM230 pamoate 11.423 % suspension are chilled for at least 15 min. Drop by drop 3.600 g G1-P/pasireotide pamoate 11.423 % suspension is added to the chitosan solution under magnetic stirring. Stirring of the obtained suspension is continued for at least 30 min or until the drug substance is thoroughly dispersed. The pH and the mean particle size (using a Lasentec probe, FBRM) is determined. The suspension is stored at 2 - 8°C.

| | Required | Actual |
|---|---|---|
| m_{CSsol} (g) | 2.400 | 2.39977 |
| m_{G1P/pasireotide pamoate 11.423%} (g) | 3.600 | 3.59806 |
| m_{CS/G1-P/Pasireotide 5%} (g) | 6.000 | 5.99783 |

### (4) Results:

| | | Required (wt %) | Actual (wt %) | pH 2-8°C |
|---|---|---|---|---|
| **Example 1** | Chitosan | 1.50 | 1.498 | |
| | G1-P | 15.00 | 15.017 | 7.30 |
| | SOM230 free base | 2.50 | 2.500 | |
| **Example 2** | Chitosan | 1.50 | 1.500 | |
| | G1-P | 15.00 | 14.996 | 7.32 |
| | SOM230 free base | 5.00 | 5.001 | |

### Peptide quantification by HPLC:

The peptide was quantified using an Agilent 1100 HPLC system (Agilent, Santa Clara, CA, USA), with a Waters symmetry shield, RP18 3.5 µm, 50 x 4.6 mm column (Waters Corporation, Milford, USA). The mobile phase A consisted of water, acetonitrile, phosphoric acid (900 : 100 : 1 V/V/V) and mobile phase B of (100 : 900 : 1 V/V/V) at a flow rate of 1.0 mL/min using a 6 steps gradient. 10 µL samples were injected at an oven temperature of 40°C. The analysis was performed by UV detection at 230 nm. All samples were injected in duplicate. The resulting chromatogram was analyzed with Chromeleon 6.8 Datasystem (Dionex Corporation, Sunnyvale, CA, USA). The reference stock solution of peptide was prepared in methanol and the standard solutions of different concentrations were prepared with release medium of pH 7.4 (see Table 2). The resulting calibration curves ranged from 3 to 250 µg/ml with a correlation coefficient above 0.999.

### In vitro release tests:

In vitro release tests were performed in triplicate using two different methods. In method I, an amount of approximately 0.5 g (accurately weighed to 0.001 g) formulation was injected via a 21G needle into a 50 mL tube and incubated 1 h in a reciprocating water bath (GFL, Burgwedel, Germany) maintained at 37°C to allow gel formation. 25 mL release medium pH 7.4 pre-warmed to 37°C was then added slowly to the depot (t = 0) and mild shaking (10%) was started. In method II, the same amount of formulation was injected directly into a 50 mL tube containing 25 mL (50 mL for the peptide-loaded formulations) release medium pH 7.4 pre-warmed to 37°C, in a reciprocating water bath (GFL, Burgwedel, Germany) maintained at 37°C (t = 0). After 1 h at rest, to allow gel formation, mild shaking (10 %) was started. For method I and II, at defined time points, the tubes were gently homogenized, centrifuged and 10 mL samples (25 mL for the peptide-loaded formulations) were removed from the media and replaced with fresh release medium to maintain the volume constant and to maintain the sink conditions. All measurements were performed in duplicate. The amount of peptide released was determined by HPLC, as described above. The composition of the release medium pH 7.4 used for each release test is listed in Table 3.

**Table 3: Composition of the release media pH 7.4 used for the in vitro release tests**

| Component | Quantity |
|---|---|
| Na₂HPO₄ | 1.09 g |
| KH₂PO4 | 0.32 g |
| NaCl | 8.06 g |
| Benzalkonium chloride | 0.01 g |
| Polysorbate 80* | 4.00 g |
| Water | qs 1000.0 mL |

| | |
|---|---|
| *: polysorbate 80 was added as surfactant to improve the peptide solubility in the release medium | |

### Sustained-release behavior of peptide-loaded CS / G1-P depot:

The CS / G1-P / peptide formulations turned into solid depots after injection into the release medium pH 7.4 at 37°C. In contrast, the peptide-loaded CS solution (without gelling agent) progressively solidified into numerous aggregates, due to the pH of the medium. The CS / G1-P/ peptide depots maintained their shape and structural integrity over the 90 days of release test.

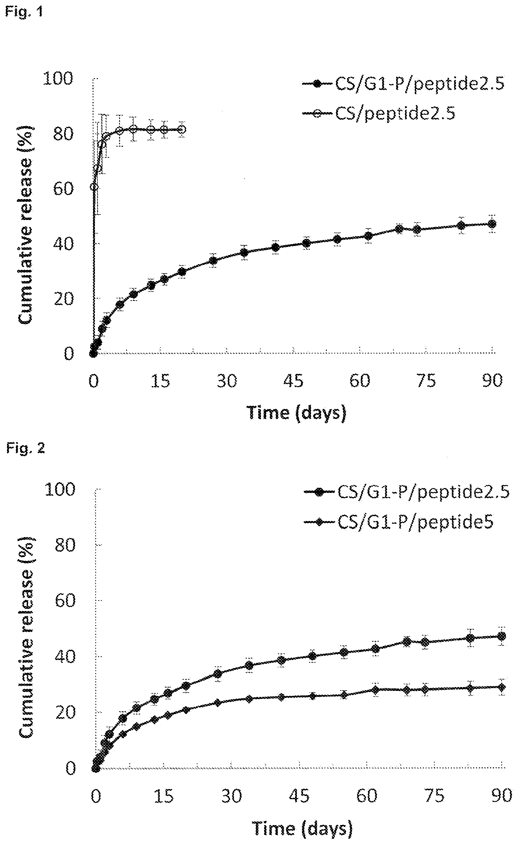

The release profile of the peptide-loaded CS / G1-P hydrogel is displayed in **Fig. 1****.**

Addition of G1-P to the CS solution significantly reduced the initial burst (2.5 ± 2.1 % release within 6 h), compared with the peptide suspension without gelling agent (60.6 ± 16.8 %). Furthermore, CS / G1 P / peptide formulation showed a sustained release of the peptide over more than 3 months, whereas 80 % released was achieved in about 3 days for the pure CS-based positive control. Actually, the depots comprising 2.5 % peptide exhibited 47 % release within 90 days, following a biphasic release pattern. It displayed a small burst of about 20 % throughout the first week, followed by a slower sustained release phase.

A small standard deviation in the quantity of peptide released, mostly below ± 3 %, was observed between the triplicate samples through the complete test period, showing that the depot formulations achieve good repeatability of the release overtime.

### Influence of drug loading:

As shown in Fig. 2, doubling the peptide loading between CS / G1-P / peptide2.5 and CS / G1-P / peptide5 formulations did not significantly affect the initial burst (2.5 % and 2.3 % respectively in 6 h) and the overall biphasic release pattern was maintained. However, during the phase of slower release, the 5 %-loaded hydrogel released the peptide slower than the 2.5 %-loaded hydrogel, leading to a difference of 18 % between the two formulations after 90 days.

The following analytical tests of the exemplified embodiments of the invention were performed with corresponding placebo formulations the results of which are equally representative to the peptide comprising formulations.

### Preparation of CS / G1-P placebo solutions:

CS solutions were obtained by dissolving 3.75 wt.% CS in HCI according to a molar ratio for CS amine groups / HCI equal to 0.9/1. G1-P solutions were prepared at various concentrations in MilliQ water. CS and G1-P solutions were separately cooled to 4°C for 15 minutes. Then, G1-P solution was added drop-by-drop into the CS solution placed in an ice-bath under magnetic stirring. The obtained CS / G1-P solution was further stirred for 15 minutes. The resulting formulations were stored at 2 - 8°C. As a reference, CS / β-glycerophosphate disodium salt hydrate (β-GP, Mw: 288.10 g/mol) solution was prepared according to the same procedure.

### Rheological measurements:

Rheological measurements were performed using a Haake Rheostress1 rheometer (Thermo Fisher Scientific, Karlsruhe) with a circulating environmental system for temperature control. The sample volume was about 0.5 mL, introduced between a cone-plate geometry (2° cone angle, 35 mm diameter). A solvent trap was used to minimize water evaporation during all tests. To determine their viscosity at 20.00 ± 0.20°C, freshly prepared degassed samples, prepared with 1.5 wt.% Chitoceutical® 90/50(1) and G1-P concentration ranging from 0.00 to 0.53 mmol/g, were subjected to rotational tests at controlled shear rates, during which the shear rate was kept constant for 60 s and then increased stepwise from 5 to 10³ s⁻¹. The apparent shear viscosity values were calculated as the mean of the apparent shear viscosities determined after 30 s equilibration at each shear rate. Following tests were performed: (i) time sweep tests, to determine gelation times, were carried out at constant angular frequency of 1 Hz and constant temperature of 37.00 ± 0.20°C. The sol / gel transition temperature (T_{gel}) or time (t_{gel}) correspond to the intersection of the curves of G' and G"; *(iii)* the strength of the hydrogels was evaluated by performing frequency sweep tests on hydrogels formed either in contact with air or in PBS. Gels were made as follows, 1 mL CS / G1-P solution were poured in round shaped molds and were incubated 6 h, 24 h and 48 h at 37°C to allow formation of hydrogels, either in an atmosphere saturated in moisture to avoid water evaporation or in 1 mL PBS pre-heated at 37°C.

### Stability testing:

Stability tests were carried out with different CS solutions: (i) a pure CS solution, 1.5 wt.% Chitoceutical® 90/50(2); two CS / G1-P solutions *(ii)* 1.5 wt.% Chitoceutical® 90/50(1) / 0.27 mmol/g G1-P and *(iii)* 1.5 wt.% Chitoceutical® 90/50(1) / 0.40 mmol/g G1-P, and *(iv)* a CS / β-GP solution, and as a reference 1.5 wt.% Chitoceutical® 90/50(1) / 0.40 mmol/g β-gtycerophosphate disodium salt hydrate (β-GP, Mw: 288.10 g/mol). These solutions were filtered through 0.22 µm filters, filled in 2R glass vials and purged with nitrogen prior to hermetic sealing. Mean headspace oxygen content determined by frequency modulated spectroscopy using the Lighthouse FMS-760 Instrument (Lighthouse Instruments, LLC) was 0.70 ± 0.07 %. Samples were stored at refrigerated conditions (i.e. 2 - 8°C) and at room temperature (i.e. 20 - 25°C), and analyzed after t = 1, 7, 14, 30, 60, 90, 135, 180 and 270 days for the samples stored at 2 - 8°C and at t = 1, 7, 14, 30, 60 and 180 days for the samples stored at 20 - 25°C respectively. At each time point, a panel of different characteristics were followed for each samples: *(i)* the formulation was visually inspected for any change in the physical appearance, i.e., color, turbidity, consistency (liquid or gel state); *(ii)* the pH value was determined using a 691 pH meter (Metrohm, Herisau, Switzerland) ; *(iii)* the viscosity at 20.00 ± 0.20°C was measured by performing a step test as described under section "Rheological measurements"; *(iv)* the gelation time was determined by a time sweep test as defined in "Rheological measurements".

### Storage stability of CS / gelling agent solutions:

Storage stability is a crucial parameter that needs to be assessed during pharmaceutical development of an *in situ* forming hydrogel system. This type of formulations should have an acceptable shelf life, i.e. their physical thermogelling properties should be maintained throughout defined storage conditions up to administration. This section presents the stability study of two CS / G1-P thermogelling solutions (with 0.27 and 0.40 mmol/g G1-P) according to the present invention in comparison with the reference CS / β-GP thermogelling solution (with 0.40 mmol/g β-GP) and pure CS solution (at the same polymer concentration: 1.5 wt.%). The study included following-up over 6 to 9 months, for two different storage temperatures (2 - 8°C and 20 - 25°C), the appearance, pH, viscosity and gelation time of the solutions as well as CS molecular weight.

### Appearance and pH:

Overall, no change in the appearance (liquid state) nor in the pH values of the CS and CS / G1-P solutions were observed over the 6 to 9 months storage (for both temperatures). The pH of the pure CS solution remained 5.2 ± 0.1 while those of CS / G1-P solutions were 7.1 ± 0.1 and 7.2 ± 0.1, for 0.27 and 0.40 mmol/g G1-P, respectively. On the contrary, CS / β-GP solution turned into a turbid gel in less than 30 days at 2 - 8°C and after only 1 day at 20 - 25°C, with a drop of the pH value from 7.4 to 7.0.

### Viscosity:

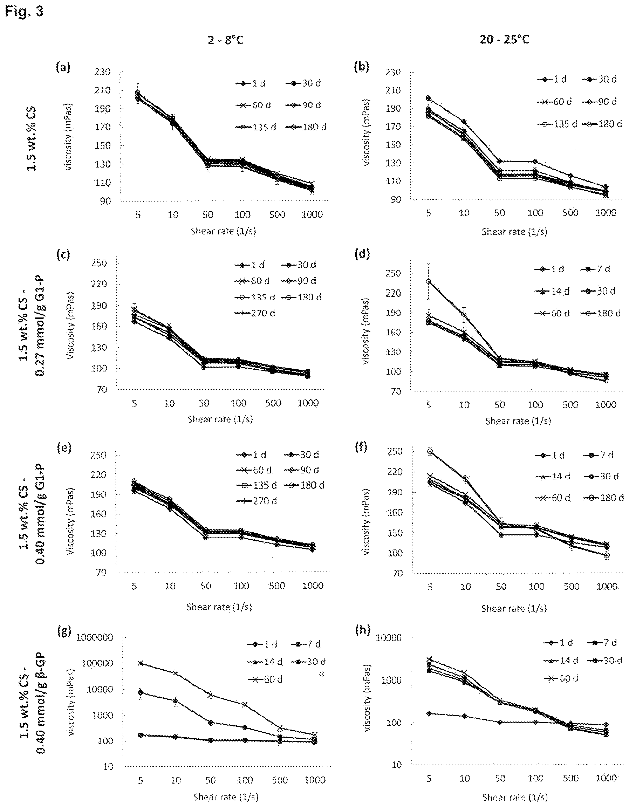

Apparent shear viscosities of the CS solution and CS / gelling agent systems are reported in Fig. 3. Results show that for storage periods as long as 6 to 9 months and at refrigerated conditions, the apparent shear viscosity of the pure CS and CS / G1-P solutions remained unchanged. On the contrary, the viscosity of CS / β-GP increased significantly within 30 days, from about 180 mPas up to 7500 mPas at a shear rate of 5 s⁻¹. Likewise, the viscosity of CS / G1-P solutions was stable at room temperature over 2 months and only slightly increases at low shear rates after 6 months, whereas the viscosity of the pure CS solution very slightly reduced over time and the viscosity of the CS / β-GP solution raised drastically after only 7 days. These results are fully aligned with former observations, i.e. the pure CS solution as well as the CS / G1-P solutions remained in the liquid state over time for both storage conditions while the CS / β-GP solution turned into gel with time. Storage under refrigerated conditions allowed maintaining the CS / β-GP formulation in the liquid state for just over 14 days versus less than 7 days at room temperature. Once the sol / gel transition occurred, the viscosity of the CS / β-GP gels continue to increase up to stabilization (more appreciable at 2 - 8 °C in Fig. 3 (g)).

### Gelation time:

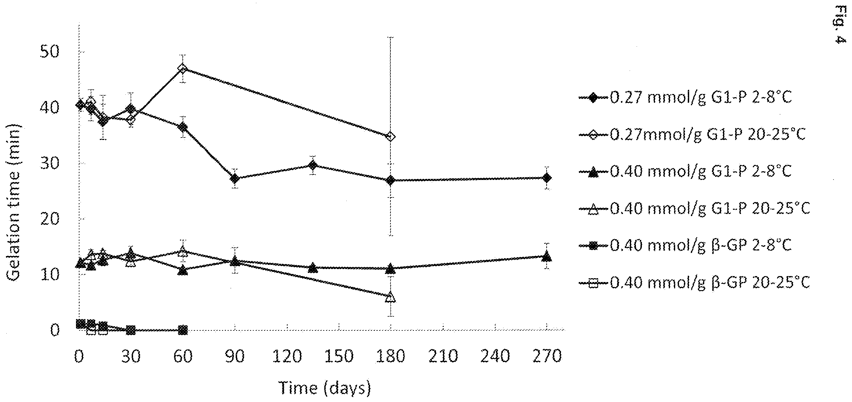

The sol / gel transition times of the different CS / gelling agent formulations set up at 37°C were followed over the same storage period and reported in Fig. 4. It appears that the gelation time of the solution composed of 0.27 mmol/g G1-P is not stable overtime, and decreases from about 40 min initially, to 35 min and 27 min after 6 months storage at 20 - 25°C and 2 - 8°C respectively, with significant variations observed over time under 20 - 25°C conditions. These variations vanish when the G1-P concentration is increased to 0.40 mmol/g. Indeed, the gelation time observed for the CS / G1-P solution containing 0.40 mmol/g G1-P stored at 20 - 25°C was stable over 2 months, but slightly decreased afterwards. However, when stored under refrigerated conditions, this solution displayed a constant gelation time of 12.2 ± 1.0 min over 9 months. With regards to the CS / β-GP solutions, the samples underwent a spontaneous gelation in less than 30 days at 2 - 8°C and 1 day at 20 - 25°C. These observations were confirmed by the rheological measurements of the gelation time, since the CS / β-GP system exhibited a gel-like behavior (G' > G") from the beginning of the time sweep test. However already before these time points, the gelation times were nevertheless very fast, in the range of 1 to 2 min. So the CS / β-GP solution was not stable when stored either at room temperature or under refrigerated conditions. Conversely, the addition of 0.40 mmol/g G1-P as alternative gelling agent to the CS solution significantly improved the stability of the system, in particular when stored at 2 - 8°C.

### Evaluation of injectability:

The injection force for a CS / G1-P solution containing 1.5 wt.% Chitoceutical® 90/50(1) and 0.40 mmol/g G1-P was measured using an electronic tensile tester (Zwick Z 2.5, Ulm, Germany) and analyzed with the testXpert II software (version 3.0, Zwick, Ulm, Germany). 1 mL thermogelling solution was injected into an empty glass vessel, using a 1 mL syringe (1 mL Luer-Lok Tip syringe, BD, Franklin Lakes, NJ, USA). Several needles were tested: *(i)* 23 G (HSW FINE-JECT® 23 G x 1", Henke-Sass Wolf GmbH, Tuttlingen, Germany), *(ii)* 25 G (Terumo® Hypodermic Needles, 25 G x 1" Thin Wall Needle, Terumo Medical Corporation, Somerset, NJ, USA) and *(iii)* 30 G (BD Microlance™ 3, 30 G x½", BD, Franklin Lakes, NJ, USA). Before measurement, all syringes were equilibrated 10 min at room temperature and primed to expel the air bubbles until a small amount of the solution came out. Injection force was measured at two different injection speeds: 80 and 100 mm/min, for each needle size. All measurements were replicated 10 times.

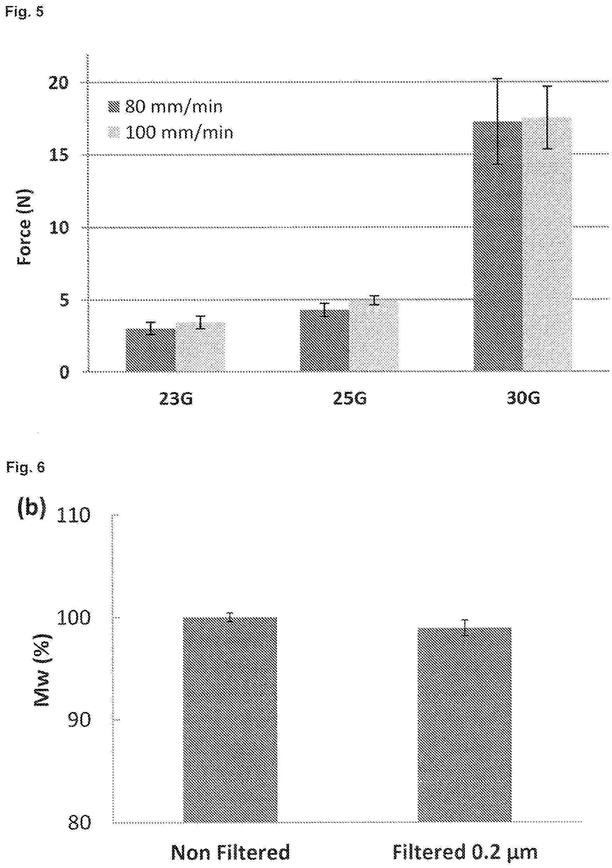

The upper limit of 20 N was set as acceptance criteria for s.c. injections, according to Schoenhammer et al in Pharmaceutical Research, 26 (2009) 2568-2577. The results are presented in Fig. 5, and show that, only the needle size significantly impacted the injection force. The force necessary to inject the CS / G1-P solution was below 5 N when using 23 G and 25 G needles, which is considered as allowing very smooth injections. Using a thinner needle of 30 G required an increased injection force of about 17.5 N, which still did not exceed the upper limit of 20 N. Therefore, the CS-based formulation of the present invention can be considered as easy to inject through needles as thin as 30 G or thinner.

### Sterile filtration:

CS / G1-P solution containing 1.5 wt.% Chitoceutical® 90/50(1) and 0.40 mmol/g G1-P were filtered through 0.22 µm syringe filters (hydrophilic, 33 mm diameter, 4.5 cm² filtration area, Millex-GV Filters, Millipore). The influence of sterile filtration on the solution characteristics was evaluated on three batches by studying the molecular weight of CS, determined by GPC, measured before and after sterile filtration. The molecular weights of the CS in the filtered solutions were calculated relative to the Mw of non-filtered solutions. All measurements were performed in triplicate.

Sterile filtration was easily performed on the CS / G1-P solution. As presented in Fig. 6, the viscosity of the solution remained unchanged after filtration. GPC analysis showed only a slight decrease of about 1 % of the molecular weight of the polymer between the non-filtered and the filtered formulation, this value being within the accuracy range of the method (± 5 %). Thus, no polymeric material is deemed to be lost on the filter during sterile filtration.

### In vivo administration / local tolerability of CS / G1-P hydrogel:

The local tolerability of CS / G1-P hydrogels over three weeks was examined in healthy 10 week old Hans Wistar male rats. 0.5 mL sterile CS / G1-P thermogelling solution (1.5 wt.% Chitoceutical® 90/50(2), 0.40 mmol/g G1-P), equilibrated at room temperature, was subcutaneously injected using a 23 G needle in the interscapular region of nine rats under general anesthesia. The same volume of sterile saline solution was subcutaneously injected in their caudal dorsal area as a negative control. The health status and body weight of the animals were monitored. Blood samples were collected under isoflurane by sublingual puncture at day -3 (predose) for hematology analysis and by vena cava puncture on the day of necropsy for hematology and biochemistry analysis. After 1, 7 and 21 days, three rats were sacrificed via exsanguination from vena cava puncture after exposure to isoflurane and the tissues at the injection sites were excised for histological examination.

**Table 4: Histological examination of s.c. tissue of rats implanted with CS / G1-P hydrogels.**

| Time point post injection | Inflammation | Fibrosis |
|---|---|---|
| 1 day | +++ / ++++ acute | - |
| 7 days | +++ / ++++ subacute | + / ++ immature |
| 21 days | ++ chronic | + / ++ mature |

| | | |
|---|---|---|
| For inflammation, "++", "+++" and "++++" indicates mild, moderate and marked inflammatory response respectively. For fibrosis, "-", "+" and "++" represents no, minimal and slight fibrosis respectively. | | |

The inflammatory reaction observed around the CS / G1-P formulation injected subcutaneously in rats was a typical foreign body reaction, similar to the tissue response reported for other depot systems like PLGA microparticles or standard CS / β-GP hydrogels. Following the injection of the formulation, acute to chronic inflammation occurred in a sequential order and inflammation tended to fade with time. Fibrosis accompanied the inflammatory response and surrounded the implant site; it started 7 days post injection with a well vascularized immature granulation tissue and towards 21 days, it formed a slightly vascularized thick capsule composed of fibroblasts and collagen fibers. Thus, the hydrogel exhibited acceptable tissue biocompatibility.

## Claims

1. A drug delivery system comprising
(a) a pharmaceutically active peptide or any pharmaceutically acceptable salt thereof,
(b) 1 to 2 % of weight of a chitosan or chitosan derivative, having a deacylation degree (DD) from 85 to 95% and a molecular weight (MW) from 100 to 200 kDa,
(c) 10 to 25 % of weight of a sugar-phosphate, and
(d) water,
based on the total weight of the drug delivery system.

2. The drug delivery system according to claim 1 wherein one said peptide is hydrophobic.

3. The drug delivery system according to any one of the preceding claims wherein one said peptide is is present in the form of a hydrophobic salt, e.g. the peptide is present as pamoate salt.

4. The drug delivery system according to any one of the preceding claims wherein one said peptide is octreotide, pasireotide, vapreotide, or lanreotide, preferably octreotide or pasireotide, more preferably pasireotide.

5. The drug delivery system according to any one of the preceding claims wherein said chitosan has a deacylation degree (DD) of 90 ± 2.5 %.

6. The drug delivery system according to any one of the preceding claims wherein said chitosan has a molecular weight (MW) from 100 to 150 kDa, preferably a MW of 135 ± 20 kDa.

7. The drug delivery system according to any one of the preceding claims wherein the sugar of said sugar-phosphate is a monosaccharide, disaccharide, or a oligosaccharide, preferably a monosaccharides selected from the group of aldoses, e.g. glucose, mannose, galactose, allose, altrose, gulose, idose, talose, ribose, arabinose, xylose, lyxose, or from the group of ketoses, e.g. fructose, sorbose, tagatose, psicose, araboketose, xyloketose, or a mixture thereof, preferably glucose or fructose or a mixture thereof.

8. The drug delivery system according to any one of the preceding claims wherein said sugar-phosphate is a glucose-phosphate, preferably a glucose-1-phosphate, preferably a alpha-D-glucose-1-phosphate, more preferably alpha-D-glucose-1-phosphate disodium hydrate.

9. The drug delivery system according to any one of the preceding claims, comprising at least 1 %, at least 2%, 1 to 30 %, 2 to 10 %, or 2 to 5 %, preferably 2 to 5 % by weight of the peptide in its free base form based on the total weight of the drug delivery system.

10. The drug delivery system according to any one of the preceding claims comprising, substantially comprising, essentially consisting of, or consisting of, preferably consisting of
(a) 2 to 5 % by weight of a hydrophobic peptide in its free base form, preferably present as pamoate salt, preferably the petide is pasireotide pamoate,
(b) 1 to 2 % by weight of a chitosan having a DD of 90 ± 2.5 % and a MW of 135 ± 20 kDa, subjected to an acid, preferably subjected to hydrochloric acid.
(c) 10 - 25 % by weight of glucose-1-phosphate, and
(d) water.
based on the total weight of the drug delivery system.

11. A process for preparing the drug delivery system as defined by any one of the claims 1 to 10 comprising following process steps:
(1) dissolving the chitosan in an acidic aqueous solution, preferably in a hydrochloric acid solution,
(2) dissolving the sugar-phosphate in an aqueous medium, preferably in water,
(3) dissolving, partly dissolving, or dispersing the peptide into the sugar-phosphate solution, preferably dispersing the peptide, and
(4) combining the peptide/glucose-phosphate solution and/or suspension with the chitosan solution.

12. A dosage form comprising the drug delivery system according to any one of the claims 1 to 10, in the form of a liquid, preferably a suspension in an ampoule, vial or pre-filled syringe, preferably in the form of a suspension in a pre-filled syringe equipped with an injection needle of a 21G or thinner, preferably 25G or thinner.

## Patentansprüche

1. Arzneimittelverabreichungssystem, umfassend:
(a) ein pharmazeutisch aktives Peptid oder ein pharmazeutisch unbedenkliches Salz davon,
(b) 1 bis 2 Gew.-% eines Chitosans oder Chitosanderivats mit einem Deacylierungsgrad (DG) von 85 bis 95% und einem Molekulargewicht (MG) von 100 bis 200 kDa,
(c) 10 bis 25 Gew.-% eines Zuckerphosphats und
(d) Wasser,
basierend auf dem Gesamtgewicht des Arzneimittelverabreichungssystems.

2. Arzneimittelverabreichungssystem nach Anspruch 1, wobei ein Peptid hydrophob ist.

3. Arzneimittelverabreichungssystem nach einem der vorhergehenden Ansprüche, wobei ein Peptid in Form eines hydrophoben Salzes vorliegt, wobei das Peptid z. B. als Pamoatsalz vorliegt.

4. Arzneimittelverabreichungssystem nach einem der vorhergehenden Ansprüche, wobei es sich bei einem Peptid um Octreotid, Pasireotid, Vapreotid oder Lanreotid, vorzugsweise Octreotid oder Pasireotid, besonders bevorzugt Pasireotid handelt.

5. Arzneimittelverabreichungssystem nach einem der vorhergehenden Ansprüche, wobei das Chitosan einen Deacylierungsgrad (DG) von 90 ± 2,5% hat.

6. Arzneimittelverabreichungssystem nach einem der vorhergehenden Ansprüche, wobei das Chitosan ein Molekulargewicht (MG) von 100 bis 150 kDa, vorzugsweise ein MG von 135 ± 20 kDa hat.

7. Arzneimittelverabreichungssystem nach einem der vorhergehenden Ansprüche, wobei es sich bei dem Zucker des Zuckerphosphats um ein Monosaccharid, Disaccharid oder Oligosaccharid handelt, vorzugsweise ein Monosaccharid ausgewählt aus der Gruppe der Aldosen, z. B. Glucose, Mannose, Galactose, Allose, Altrose, Gulose, Idose, Talose, Ribose, Arabinose, Xylose, Lyxose, oder aus der Gruppe der Ketosen, z. B. Fructose, Sorbose, Tagatose, Psicose, Araboketose, Xyloketose, oder um eine Mischung davon, vorzugsweise Glucose oder Fructose oder eine Mischung davon.

8. Arzneimittelverabreichungssystem nach einem der vorhergehenden Ansprüche, wobei es sich bei dem Zuckerphosphat um ein Glucosephosphat, vorzugsweise ein Glucose-1-phosphat, bevorzugt alpha-D-Glucose-1-phosphat, besonders bevorzugt alpha-D-Glucose-1-phosphat-dinatrium-hydrat, handelt.

9. Arzneimittelverabreichungssystem nach einem der vorhergehenden Ansprüche, welches mindestens 1 Gew.-%, mindestens 2 Gew.-%, 1 bis 30 Gew.-%, 2 bis 10 Gew.-% oder 2 bis 5 Gew.-%, vorzugsweise 2 bis 5 Gew.-% des Peptids in Form seiner freien Base umfasst, basierend auf dem Gesamtgewicht des Arzneimittelverabreichungssystems.

10. Arzneimittelverabreichungssystem nach einem der vorhergehenden Ansprüche umfassend, im Wesentlichen umfassend, im Wesentlichen bestehend aus oder bestehend aus, vorzugsweise bestehend aus
(a) 2 bis 5 Gew.-% eines hydrophoben Peptids in Form seiner freien Base, vorzugsweise als Pamoatsalz vorliegend, wobei es sich bei dem Peptid bevorzugt um Pasireotidpamoat handelt,
(b) 1 bis 2 Gew.-% eines Chitosans mit einem DG von 90 ± 2,5% und einem MG von 135 ± 20 kDa, einer Säure ausgesetzt, vorzugsweise Salzsäure ausgesetzt,
(c) 10 - 25 Gew.-% Glucose-1-phosphat und
(d) Wasser,
basierend auf dem Gesamtgewicht des Arzneimittelverabreichungssystems.

11. Verfahren zur Herstellung des durch einen der Ansprüche 1 bis 10 definierten Arzneimittelverabreichungssystems, welches die folgenden Verfahrensschritte umfasst:
(1) Lösen des Chitosans in einer sauren wässrigen Lösung, vorzugsweise in einer Salzsäurelösung,
(2) Lösen des Zuckerphosphats in einem wässrigen Medium, vorzugsweise in Wasser,
(3) Lösen, teilweises Lösen oder Dispergieren des Peptids in der Zuckerphosphatlösung, vorzugsweise Dispergieren des Peptids, und
(4) Kombinieren der Peptid/Glucosephosphat-Lösung und/oder -Suspension mit der Chitosanlösung.

12. Dosierungsform, umfassend das Arzneimittelverabreichungssystem nach einem der Ansprüche 1 bis 10 in Form einer Flüssigkeit, vorzugsweise einer Suspension, in einer Ampulle, einem Vial oder einer vorgefüllten Spritze, vorzugsweise in Form einer Suspension in einer mit einer 21G- oder dünneren Injektionsnadel, vorzugsweise 25G- oder dünneren Injektionsnadel ausgestatteten vorgefüllten Spritze.

## Revendications

1. Système de libération de médicament comprenant
(a) un peptide pharmaceutiquement actif ou n'importe quel sel pharmaceutiquement acceptable de celui-ci,
(b) 1 à 2 % en masse d'un chitosane ou d'un dérivé de chitosane, présentant un degré de désacylation (DD) compris entre 85 et 95 % et une masse moléculaire (MW) comprise entre 100 et 200 kDa,
(c) 10 à 25 % en masse d'un sucre-phosphate, et
(d) de l'eau,
par rapport à la masse totale du système de libération de médicament.

2. Système de libération de médicament selon la revendication 1, où ledit peptide est hydrophobe.

3. Système d'administration de médicament selon l'une quelconque des revendications précédentes, où ledit peptide est présent sous la forme d'un sel hydrophobe, par exemple, le peptide est présent sous forme d'un sel de pamoate.

4. Système de libération de médicament selon l'une quelconque des revendications précédentes, où ledit peptide est l'un des suivants : octréotide, pasiréotide, vapréotide ou lanréotide, préférentiellement octréotide ou pasiréotide, plus préférentiellement pasiréotide.

5. Système d'administration de médicament selon l'une quelconque des revendications précédentes, où ledit chitosane présente un degré de désacylation (DD) de 90 ± 2,5 %.

6. Système de libération de médicament selon l'une quelconque des revendications précédentes, où ledit chitosane présente une masse moléculaire (MW) comprise entre 100 et 150 kDa, préférentiellement une MW de 135 ± 20 kDa.

7. Système de libération de médicament selon l'une quelconque des revendications précédentes, où le sucre dudit sucre-phosphate est un monosaccharide, un disaccharide ou un oligosaccharide, préférentiellement un monosaccharide choisi dans le groupe constitué par les aldoses, par exemple glucose, mannose, galactose, allose, altrose, gulose, idose, talose, ribose, arabinose, xylose, lyxose, ou dans le groupe constitué par les cétoses, par exemple fructose, sorbose, tagatose, psicose, arabocétose, xylocétose, ou l'un de leurs mélanges, préférentiellement glucose ou fructose ou l'un de leurs mélanges.

8. Système de libération de médicament selon l'une quelconque des revendications précédentes, où ledit sucre-phosphate est un glucose-phosphate, préférentiellement un glucose-1-phosphate, préférentiellement un alpha-D-glucose-1-phosphate, plus préférentiellement l'alpha-D-glucose-1-phosphate disodium hydraté.

9. Système de libération de médicament selon l'une quelconque des revendications précédentes, comprenant au moins 1 %, au moins 2 %, 1 à 30 %, 2 à 10 % ou 2 à 5 %, préférentiellement 2 à 5 % en masse du peptide sous sa forme de base libre, par rapport à la masse totale du système de libération de médicament.

10. Système de libération de médicament selon l'une quelconque des revendications précédentes comprenant, comprenant substantiellement, essentiellement constitué de, ou constitué de, préférentiellement constitué de
(a) 2 à 5 % en masse d'un peptide hydrophobe sous sa forme de base libre, préférentiellement présent sous forme de sel de pamoate, préférentiellement, le peptide est le pamoate de pasiréotide,
(b) 1 à 2 % en masse d'un chitosane présentant un DD de 90 ± 2,5 % et une MW de 135 ± 20 kDa, soumis à un acide, préférentiellement soumis à l'acide chlorhydrique,
(c) 10 à 25 % en masse de glucose-1-phosphate, et
(d) de l'eau,
par rapport à la masse totale du système de libération de médicament.

11. Procédé d'élaboration du système de libération de médicament selon l'une quelconque des revendications 1 à 10, comprenant les étapes de procédé suivantes :
(1) dissolution du chitosane dans une solution aqueuse acide, préférentiellement dans une solution d'acide chlorhydrique,
(2) dissolution du sucre-phosphate dans un milieu aqueux, préférentiellement dans l'eau,
(3) dissolution, dissolution partielle ou dispersion du peptide dans la solution de sucre-phosphate, préférentiellement dispersion du peptide, et
(4) combinaison de la solution et/ou la suspension de peptide/glucose-phosphate avec la solution de chitosane.

12. Forme galénique comprenant le système de libération de médicament selon l'une quelconque des revendications 1 à 10, sous la forme d'un liquide, préférentiellement d'une suspension dans une ampoule, un flacon ou une seringue préremplie, préférentiellement sous la forme d'une suspension dans une seringue préremplie équipée d'une aiguille à injection de 21G ou plus fine, préférentiellement 25G ou plus fine.
